(19) Europäisches Patentamt
European Patent Office
Office européen des brevets

(11) **EP 3 848 891 B1**

(12) **EUROPEAN PATENT SPECIFICATION**

(45) Date of publication and mention
of the grant of the patent:
**22.02.2023 Bulletin 2023/08**

(21) Application number: **19893880.5**

(22) Date of filing: **17.09.2019**

(51) International Patent Classification (IPC):
**G06T 7/246** *(2017.01)*      *A61B 1/00* *(2006.01)*
*A61B 5/00* *(2006.01)*      *A61B 1/31* *(2006.01)*

(52) Cooperative Patent Classification (CPC):
**A61B 1/00009; A61B 1/00045; A61B 1/00055;**
**A61B 1/31; A61B 5/0077; A61B 5/4255;**
**A61B 5/4836; A61B 5/486; A61B 5/747;**
**G06T 7/246;** G06T 2207/10068; G06T 2207/30028

(86) International application number:
**PCT/CN2019/106102**

(87) International publication number:
**WO 2020/114037 (11.06.2020 Gazette 2020/24)**

(54) **COMPUTER VISION-BASED METHOD AND SYSTEM FOR MONITORING SPEED OF INTESTINAL LENS REMOVAL IN REAL TIME**

AUF COMPUTERSICHT BASIERTES VERFAHREN UND SYSTEM ZUR ÜBERWACHUNG DER GESCHWINDIGKEIT DER ENTFERNUNG EINER INTESTINALEN LINSE IN ECHTZEIT

PROCÉDÉ ET SYSTÈME BASÉS SUR LA VISION ARTIFICIELLE POUR SURVEILLER LA VITESSE DE RETRAIT DE LENTILLE INTESTINALE EN TEMPS RÉEL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **05.12.2018 CN 201811481234**

(43) Date of publication of application:
**14.07.2021 Bulletin 2021/28**

(73) Proprietor: **Wuhan Endoangel Medical Technology Co., Ltd.**
**Wuhan, Hubei 430014 (CN)**

(72) Inventors:
• **YU, Honggang**
  **Wuhan, Hubei 430014 (CN)**
• **LIU, Bin**
  **Wuhan, Hubei 430014 (CN)**
• **HU, Shan**
  **Wuhan, Hubei 430014 (CN)**
• **WU, Lianlian**
  **Wuhan, Hubei 430014 (CN)**

(74) Representative: **Niburska, Danuta**
**Kancelaria Patentowa**
**Al. 3 Maja 68 B**
**76-200 Slupsk (PL)**

(56) References cited:
**WO-A1-2017/201494**

• **FILIP DOBROMIR ET AL: "Colometer: A real-time quality feedback system for screening colonoscopy", WORLD JOURNAL OF GASTROENTEROLOGY, vol. 18, no. 32, 1 January 2012 (2012-01-01), pages 4270-4277, XP055862817, CN ISSN: 1007-9327, DOI: 10.3748/wjg.v18.i32.4270 Retrieved from the Internet: URL:https://www.wjgnet.com/1007-9327/full/ v18/i32/4270.htm>**
• **GAO XUEXIN G ET AL: "Su1346 Colonoscopy Withdrawal Velocity and Image Clarity Measurement As a Novel Patient-Centric Real-Time Quality Indicator for Screening Colonoscopy", GASTROINTESTINAL ENDOSCOPY, vol. 75, no. 4, 1 April 2012 (2012-04-01), XP028920556, ISSN: 0016-5107, DOI: 10.1016/J.GIE.2012.03.771**

**Description**

The disclosure belongs to the field of information technology, and more particularly relates to a method and device for monitoring a colonoscopy withdrawal speed.

Colonoscopy is the most common method of screening for lower gastrointestinal lesions such as colorectal polyps and tumors. Colonoscopy withdrawal time refers to the actual time from the insertion of the colonoscope to the cecum to the withdrawal of the colonoscope to the anal canal, excluding the time taken for maneuvers such as staining or biopsy. Studies have shown that, as the withdrawal time increases, the rate of detection of polyps, the rate of detection of adenomas, and the average number of polyps found in patients increase significantly [1,2]. Therefore, in the guidelines for colonoscopy all over the world, the withdrawal time is considered as an important indicator of the quality of colonoscopy. The American Guidelines recommended a colonoscopy withdrawal time of 6-10 minutes. It was recommended in 2015 Guidelines for the Screening and Endoscopy of Early Colorectal Cancer in China that the withdrawal time should be not less than 6 minutes. However, although the Guidelines clearly defined the withdrawal time, in actual clinical practice, due to the lack of effective supervision and practical monitoring tools, and the large number of patients in China, the quality of endoscopy often failed to meet the standards stipulated in the Guidelines. A study showed that the colonoscopy withdrawal time was mostly 2-6 minutes, far shorter than the withdrawal time defined in the Guidelines, which seriously affects the health of patients in China [3]. Therefore, it is very important to ensure the withdrawal time under the existing technical conditions to improve the quality of colonoscopy.

To improve the quality of colonoscopy, as of October 2018, the National Health Commission of the PRC has established 18 provincial and 21 prefecture-level endoscopy quality control centers nationwide to regularly supervise the quality of endoscopy in various hospitals. In some hospitals, doctors' withdrawal time would be regularly recorded, counted and fed back to the doctors and management personnel. However, on one hand, such a monitoring mechanism can monitor the quality of colonoscopy only within a certain period of time or for part of samples, instead of all examinations; on the other hand, since it is required in the Guidelines that the total withdrawal time should be more than 6 minutes, to meet this standard, some doctors withdraw the colonoscope quickly at the beginning of colonoscopy and slow down at the ending of colonoscopy, resulting in insufficient observation of the distal colon and unnecessary waste of time on the proximal colon. Therefore, it is expected to provide a method for real-time monitoring the colonoscopy withdrawal speed based on the existing technology, to remind doctors to always control the withdrawal speed within a safe range in daily work, in order to improve the quality of colonoscopy.

"Colometer: a real-time quality feedback system for screening colonoscopy", World Journal of Gastroenterology, Vol. 18, No. 32, pages 4270-4277, recites a computer-based system and method that detects the level of image clarity, withdrawal velocity, and level of the bowel preparation in a real-time fashion from live video signal. In this method, the dynamics of moving objects in a video sequence can be assessed by frame differencing techniques; an overall minimum and maximum values of velocity changes for all 14 videos are determined in real time with 30 ms time delay, and two different threshold ranges between these values are selected corresponding to an adequate and rapid withdrawal speeds; and text indicators "adequate speed", "rapid withdrawal", and "blurry" are embedded into the video in real-time to provide a visual feedback to the endoscopist.

In addition, "Su1346 colonoscopy withdrawal velocity and image clarity measurement as a novel patient-centric real-time quality indicator for screening colonoscopy", Gastrointestinal Endoscopy, Vol. 75, No. 4, recites a software-based system or method that detects the levels of image clarity and withdrawal velocity in a real-time fashion from live video signal. In this method, threshold levels of image blurriness and the withdrawal velocity below which the visualization could be considered adequate have initially been determined arbitrarily by review of sample colonoscopy videos by two experienced endoscopists, and subsequently, an overall colonoscopy quality rating was computed based on the percentage of the withdrawal time with adequate visualization.

In addition, WO 2017/201494 A1 recites a method for polyp detection, the method acquires images from the imaging device and converts the images to greyscale images, plots position of the imaging device, computes scan speed for each position, and integrates scan speed with position plot.

A technical problem to be solved in the disclosure is to provide a colonoscopy withdrawal speed real-time monitoring method and system based on computer vision, to proactively prompt physicians for the withdrawal speed and stability and remind the doctors in case of any over-speed or instability, in order to remind the physicians to always control the withdrawal speed within a safe range and thus to improve the quality of colonoscopy.

To achieve the objective, the disclosure provides a method for monitoring a colonoscopy withdrawal speed based on computer vision, the method comprising:

1) acquiring, by endoscopic equipment, a real-time video of a colonoscopy, decoding the video into images, cropping the images, and down-sampling the cropped images wherein structural information of the images is retained;

2) converting the down-sampled images comprising the structural information to grayscale images;

3) obtaining Hash fingerprints of the images, where the Hash fingerprints are obtained through a Hash algorithm;

4) calculating a Hamming distance between the Hash fingerprint of a current greyscale image of the colonoscopy and the Hash fingerprint of each of n immediately previous grayscale images of the colonoscopy;

5) comparing the Hash fingerprints of the current greyscale image of the colonoscopy with the n immediately previous greyscale images of the colonoscopy using the corresponding calculated Hamming distances, to obtain an overlapping rate of the current greyscale image of the colonoscopy with any one of the n immediately previous greyscale images of the colonoscopy, thereby obtaining a similarity between the current greyscale image of the colonoscopy and the any one of the n immediately previous greyscale images of the colonoscopy;

6) calculating a weighted similarity of the greyscale images at a point in time t for the current grayscale image of the colonoscopy as a weighted sum of the similarity between the current greyscale image of the colonoscopy and each of the n immediately previous grayscale images of the colonoscopy;

7) converting the weighted similarity of the greyscale images at the point in time t for the current grayscale image of the colonoscopy into a stability coefficient;

8) calculating a mean stability coefficient within a period of time 0-t, wherein the mean stability coefficient is the mean of stability coefficients at all points in time;

9) analyzing the real-time video of a plurality of colonoscopies to obtain a first boundary between a standard colonoscopy video and a sub-standard colonoscopy video and a second boundary between the sub-standard colonoscopy video and a low-quality colonoscopy video, respectively; wherein the plurality of colonoscopies comprises standard colonoscopy videos, sub-standard colonoscopy videos, and low-quality colonoscopy video; the standard colonoscopy videos are colonoscopy videos with a withdrawal time of more than 6 minutes, the sub-standard colonoscopy videos are colonoscopy videos with a withdrawal time of 4-6 minutes, and the low-quality colonoscopy videos are colonoscopy videos with a withdrawal time of less than 4 minutes; and

10) monitoring in real time, according to the operations 1) to 8), a stability coefficient of a withdrawal by a physician performing the colonoscopy, and feeding the stability coefficient to the physician, wherein: when the stability coefficient is less than the first boundary, indicating the withdrawal speed of the colonoscopy is within a normal range; when the stability coefficient is greater than the first boundary, indicating the withdrawal speed of the colonoscopy is not within the normal range; when the stability coefficient is between the first boundary and the second boundary, a warning signal is given; and when the stability coefficient is greater than the second boundary, an emergency alarm is given.

In 1), the cropped images are down-sampled by bicubic (cubic convolution) interpolation.

In 2), the down-sampled images are converted to grayscale images by the following equation: $Gray = 0.30 \times R + 0.59 \times G + 0.11 \times B$, where R, G and B represent information values of red color, green color and blue color, respectively,

In 3), the Hash fingerprints of the grayscale images are obtained by difference Hashing (dHash).

In 5), the overlapping rate of the current grayscale image of the colonoscopy with any one of the n immediately previous grayscale images of the colonoscopy is calculated by the following equation: $Sim = \frac{100 \times (64 - d(x,y))}{64}$, where d(x, y) represents the Hamming distance between different greyscale images, $d(x,y) = \Sigma\, x \oplus y$, x and y represent character strings corresponding to different greyscale images, respectively, referring to the Hash fingerprints of the greyscale images, and $\oplus$ represents exclusive OR.

In 7), the weighted similarity of the greyscale images at the point in time t for the current grayscale image of the colonoscopy is converted into a stability coefficient by the following equation: $ESim = 100 - \overline{Sim}$, where $\overline{Sim}$ represents the weighted similarity of the greyscale images at the point in time t for the current grayscale image of the colonoscopy.

The disclosure further provides a device for monitoring a colonoscopy withdrawal speed based on computer vision, the device comprising:

an image acquisition module configured to acquire, by endoscopic equipment, a real-time video of a colonoscopy, decode the video into images, crop the images, and down-sample the cropped images where the structural information of the images is retained;

a grayscale image conversion module configured to convert the down-sampled images comprising the structural information to grayscale images;

a Hash fingerprint acquisition module configured to obtain the Hash fingerprints of the grayscale images, wherein the Hash fingerprints are obtained through a Hash algorithm;

a Hamming distance calculation module configured to calculate the Hamming distance between different Hash fingerprint of a current greyscale image of the colonoscopy and the Hash fingerprint of each of n immediately previous grayscale images of the colonoscopy;

a similarity calculation module configured to compare the Hash fingerprints of the current greyscale image of the colonoscopy with the n immediately previous greyscale images of the colonoscopy using the corresponding calculated Hamming distances, to obtain an overlapping rate of the current greyscale image of the colonoscopy with any one of the n immediately previous greyscale images of the colonoscopy, thereby obtaining a similarity between the current greyscale image of the colonoscopy and the any one of the n immediately previous greyscale images of the colonoscopy;

a weighted similarity calculation module configured to calculate the weighted similarity of the greyscale images at a point in time t for the current grayscale image of the colonoscopy as a weighted sum of the similarity between the current grayscale image of the colonoscopy and each of the n immediately previous grayscale images of the colonoscopy;

a stability coefficient conversion module configured to convert the weighted similarity of the greyscale images at the point in time t for the current grayscale image of the colonoscopy into a stability coefficient;

a mean stability coefficient conversion module configured to calculate a mean stability coefficient within a period of time 0-t, wherein the mean stability coefficient is the mean of stability coefficients at all points in time;

a colonoscopy video analysis module configured to analyze the real-time video of a plurality of colonoscopies to obtain a first boundary between a standard colonoscopy video and a sub-standard colonoscopy video and a second boundary between the sub-standard colonoscopy video and a low-quality colonoscopy video, respectively; wherein the plurality of colonoscopies comprises standard colonoscopy videos, sub-standard colonoscopy videos, and low-quality colonoscopy video; the standard colonoscopy videos are colonoscopy videos with a withdrawal time of more than 6 minutes, the sub-standard colonoscopy video are colonoscopy videos with a withdrawal time of 4-6 minutes, and the low-quality colonoscopy videos are colonoscopy videos with a withdrawal time of less than 4 minutes; and

a withdrawal speed feedback module configured to monitor in real time a stability coefficient of a withdrawal by a physician performing the colonoscopy, and feed the stability coefficient to the physician, wherein: when the stability coefficient is less than the first boundary, indicating the withdrawal speed of the colonoscopy is within a normal range; when the stability coefficient is greater than the first boundary, indicating the withdrawal speed of the colonoscopy is not within the normal range; when the stability coefficient is between the first boundary and the second boundary, a warning signal is given; and when the stability coefficient is greater than the second boundary, an emergency alarm is given.

Further, in the similarity calculation module, the overlapping rate of the current greyscale image of the colonoscopy with any one of the n immediately previous greyscale images of the colonoscopy is calculated by the following equation: $Sim = \frac{100 \times (64 - d(x,y))}{64}$ , where d(x, y) represents the Hamming distance between different greyscale images, d(x, y) = $\Sigma$ x $\oplus$ y, x and y represent character strings corresponding to different greyscale images, respectively, referring to the Hash fingerprints of the greyscale images, and $\oplus$ represents exclusive OR.

Further, in the stability coefficient conversion module, the weighted similarity of the greyscale images at the point in time t for the current grayscale image of the colonoscopy is converted into a stability coefficient by the following equation: $ESim = 100 - \overline{Sim}$, where $\overline{Sim}$ represents the weighted similarity of the greyscale images at the point in time t for the current grayscale image of the colonoscopy.

The following advantages are associated with the method and device for monitoring a colonoscopy withdrawal speed: by analyzing the stability of colonoscopy images at an instantaneous moment or within a period of time, the colonoscopy withdrawal speed is reflected in real time to remind doctors to control the withdrawal speed within the safe range during colonoscopy, thus improving the effectiveness of colonoscopy.

FIG. 1 is a flowchart of a method for monitoring a colonoscopy withdrawal speed according to one embodiment of the disclosure;

FIG. 2 shows the principle of down-sampling images by bicubic interpolation;

FIG. 3 is a schematic view of a closest mapping point, in the original image, of a pixel (x, y) in a target interpolated graph;

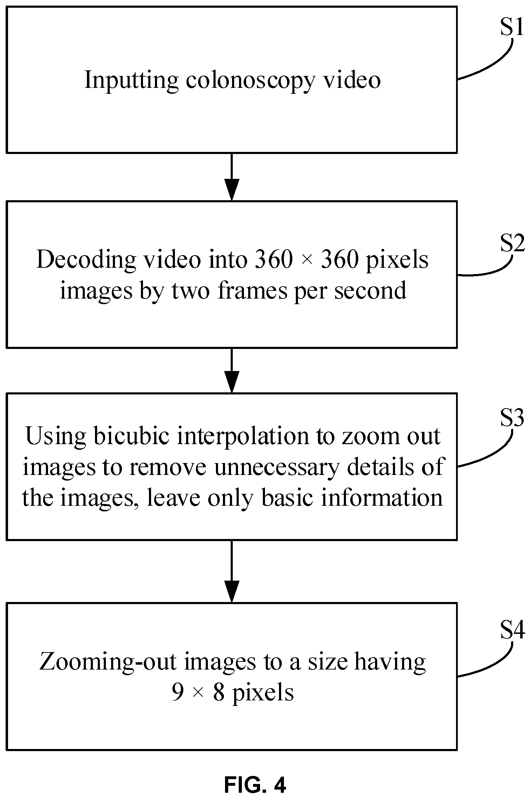

FIG. 4 is a flowchart of S1) of Example 1 of the disclosure;

FIG. 5A is a schematic view of converting the 10th and 11th images to Hash fingerprints in S1) to S3) of Example 2 of the disclosure;

FIG. 5B is a schematic view of converting the 12th and 13th images to Hash fingerprints in S1) to S3) of Example 2 of the disclosure;

FIG. 5C is a schematic view of converting the 14th and 15th images to Hash fingerprints in S1) to S3) of Example 2 of the disclosure;

FIG. 5D is a schematic view of converting the 16th and 17th images to Hash fingerprints in S1) to S3) of Example 2 of the disclosure; and

FIG. 5E is a schematic view of converting the 18th and 19th images to Hash fingerprints in S1) to S3) of Example 2 of the disclosure.

To further illustrate the invention, embodiments detailing a method and device for monitoring a colonoscopy withdrawal speed are described below. It should be noted that the following embodiments are intended to describe and not to limit the disclosure.

Example 1

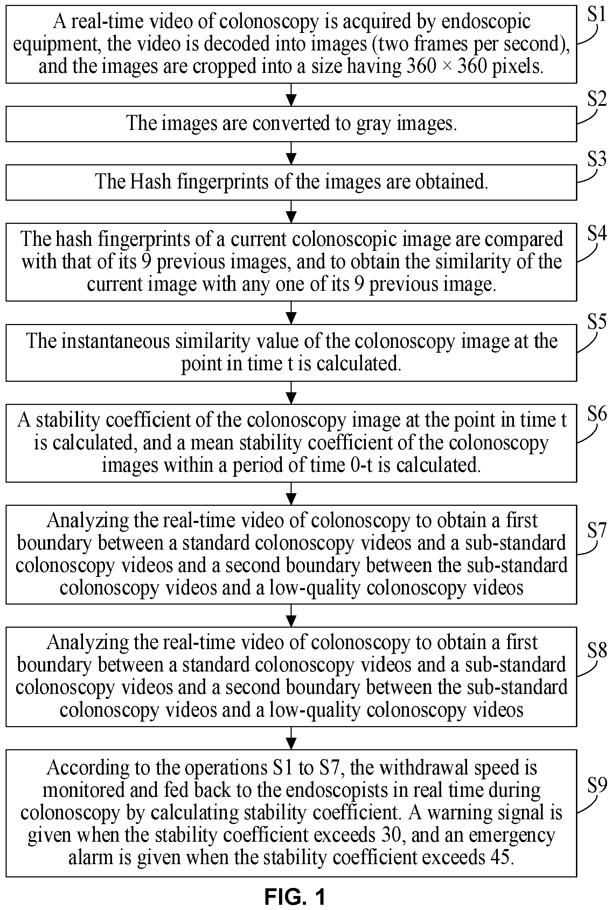

Referring to FIG. 1, the disclosure provides a method for monitoring a colonoscopy withdrawal speed, which is detailed as follows.

S1: A real-time video of colonoscopy is acquired by endoscopic equipment, the video is decoded into images (two frames per second), the images are cropped into a size having 360 × 360 pixels, and the cropped images are further down-sampled wherein structural information of the images is retained.

A 360 × 360 image has more than 100,000 pixels and contains a huge amount of information and lots of details. Therefore, it is necessary to down-sample the image to remove the unnecessary details of the images, leave only basic information such as structure, brightness and darkness, and discard the differences in images caused by different sizes and proportions.

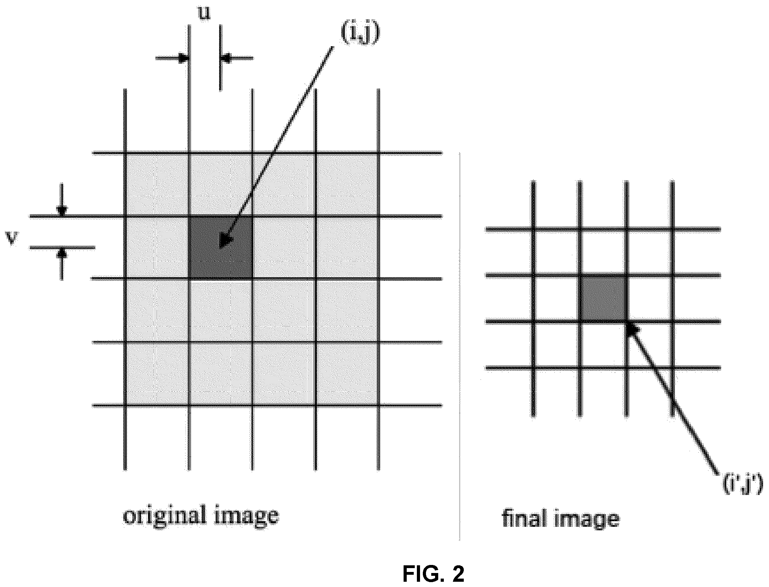

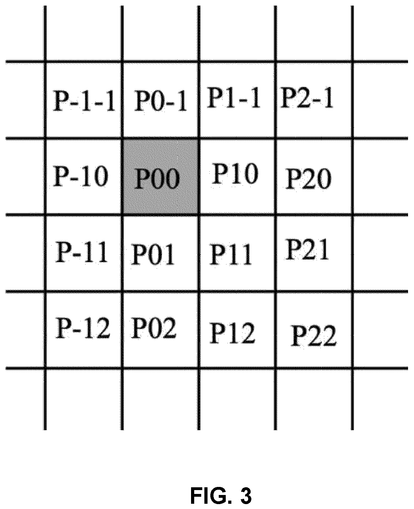

The images are down-sampled by bicubic (cubic convolution) interpolation. The down-sampled images are high in quality and less distorted, in spite of heavy calculation burden. As shown in FIG. 2 and the mathematical expression for bicubic interpolation that, the pixel value corresponding to the coordinate (i', j') in the down-sampled image after interpolation is the convolution sum of weights of 16 pixels adjacent to the coordinate (i, j) in the original image. P00 in FIG. 3 represents the closest mapping point, in the original image, of a pixel (x, y) in a target interpolated graph. Let the expression of the pixel value of each coordinate $(i, j)$ in the original image be $f(i, j)$, then the pixel value of the corresponding coordinate after interpolation is $F(i', j')$, which can be obtained by the following equation:

$$F(i', j') = \sum_{row = -1}^{2} \sum_{col = -1}^{2} f(i + row, j + col) S(row - v) S(col - u) \quad (1),$$

where v represents the deviation of the number of rows and u represents the deviation of the number of columns; row represents a certain row and col represents a certain column; $S(x)$ represents the interpolation expression which may be selected according to actual requirements, commonly including triangle interpolation, Bell interpolation and B spline interpolation. In this embodiment of the disclosure, Bell interpolation is used.

To calculate the dHash value of the images better, in this embodiment of the disclosure, the images are down-sampled to a size having 9 × 8 pixels, i.e., total 72 pixels.

S2: The images are converted to grayscale images. Usually, if the similarity of the contrast images is less related to color, the images are converted to grayscale images to decrease the complexity in the subsequent calculations. Weighted averaging is used: since people have different sensitivities to red color, green color and blue color, a different weight is provided for each pixel in the images to obtain the gray value of this pixel:

$$Gray = 0.30 \times R + 0.59 \times G + 0.11 \times B \quad (2).$$

S3: The Hash fingerprints of the images are obtained. That is, the Hash strings corresponding to the images are obtained. The common perceptual hash algorithms include aHash, pHash and dHash. aHash (average hashing) is fast, but often low in accuracy; pHash (perception hashing) is high in accuracy, but relatively slow; and dHash (difference hashing) is high in accuracy and also fast. Therefore, in this embodiment of the disclosure, the Hash fingerprints of the images are obtained by dHash.

S4: The Hamming distance between different images is calculated. In the information theory, the Hamming distance represents the number of different characters in the corresponding position of two equal-length strings. The Hamming distance between the strings x and y is denoted by d(x, y),

$$d(x, y) = \sum x \oplus y \quad (3)$$

where $\oplus$ represents exclusive OR. From another prospective, the Hamming distance measures the minimum number of replacements needed to change the string x to the character string y by means of character replacement. The Hamming distance indicates how many steps are needed to change A to B. For example, for strings "abc" and "ab3", the Hamming distance is 1, since it is just needed to change "c" to "3".

The Hamming distance in dHash is the number of differences to be changed. The differences are denoted by 0 and 1, which can be considered as binary. For binary 0110 and 1111, the Hamming distance is 2. The dhash values of the two images are converted to binary differences which are then subject to exclusive OR. The number of "1" in the result of the exclusive OR operation, i.e., the number of different digits, is counted. It is the Hamming distance.

S5: The Hash fingerprints of a current colonoscopy image with the Hash fingerprints of 9 previous colonoscopy images are compared, to obtain an overlapping rate of the current image with any one of the 9 images, i.e., the similarity between the current colonoscopy image and any one of the 9 images: $Sim = \frac{100 \times (64 - d(x,y))}{64}$.

S6: The weighted similarity of the images at a point in time t is calculated:

$\overline{Sim} = \sum_{i=1}^{9} \frac{i}{45} \times Sim_i$ where $Sim_i$ represents the similarity between the current image and the $i^{th}$ image (i ranges from 1 to 9) before the current image at the point in time t.

S7: The weighted overlapping rate at the point in time t is converted into a stability coefficient: $ESim = 100 - \overline{Sim}$.

S8: A mean stability coefficient of the colonoscopy images within a period of time 0-t is calculated, wherein the mean stability coefficient is the mean of stability coefficients at all points in time.

S9: 50 standard colonoscopy videos with a withdrawal time of more than 6 minutes, 50 sub-standard colonoscopy videos with a withdrawal time of 4-6 minutes, and 50 low-quality colonoscopy videos with a withdrawal time of less than 4 minutes are analyzed to obtain the following result: $level = \begin{cases} \overline{ESim} \leq 30 \\ 30 < \overline{ESim} < 45 \\ \overline{ESim} \geq 45 \end{cases}$.

S10: According to the operations S1 to S8, the stability coefficient of the withdrawal by a physician performing the colonoscopy is monitored in real time and fed back to the physician, a warning signal is given when the withdrawal speed exceeds 30, and an emergency alarm is given when the withdrawal speed exceeds 45.

Example 2

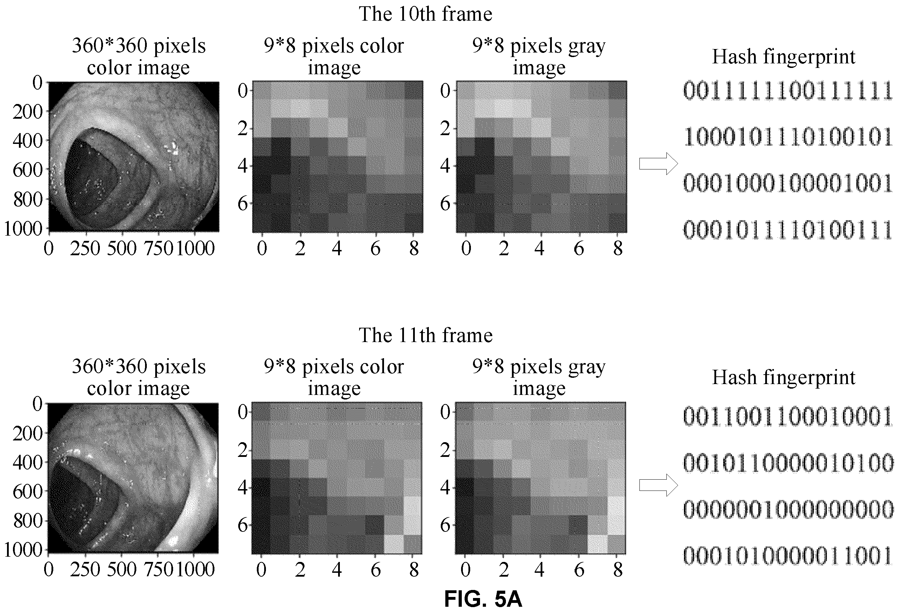

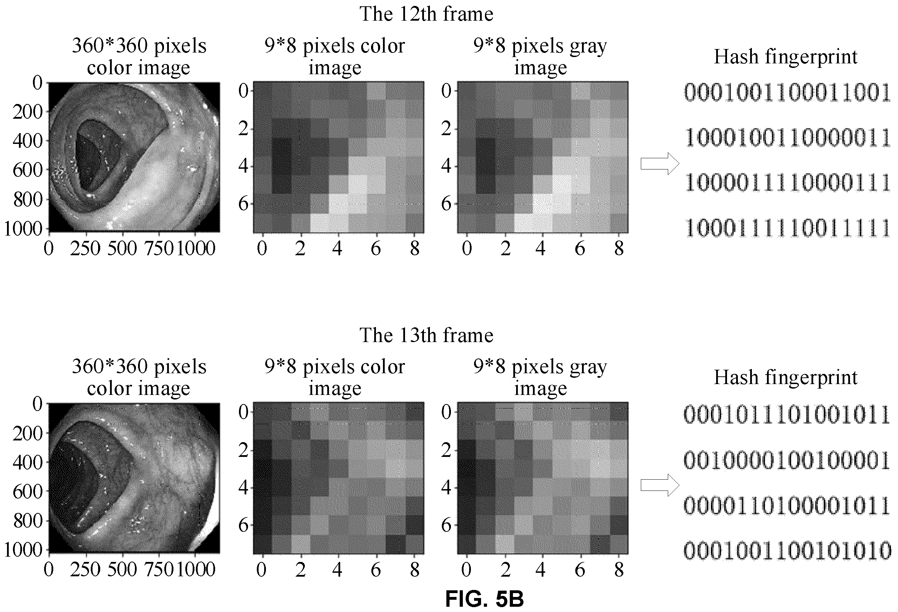

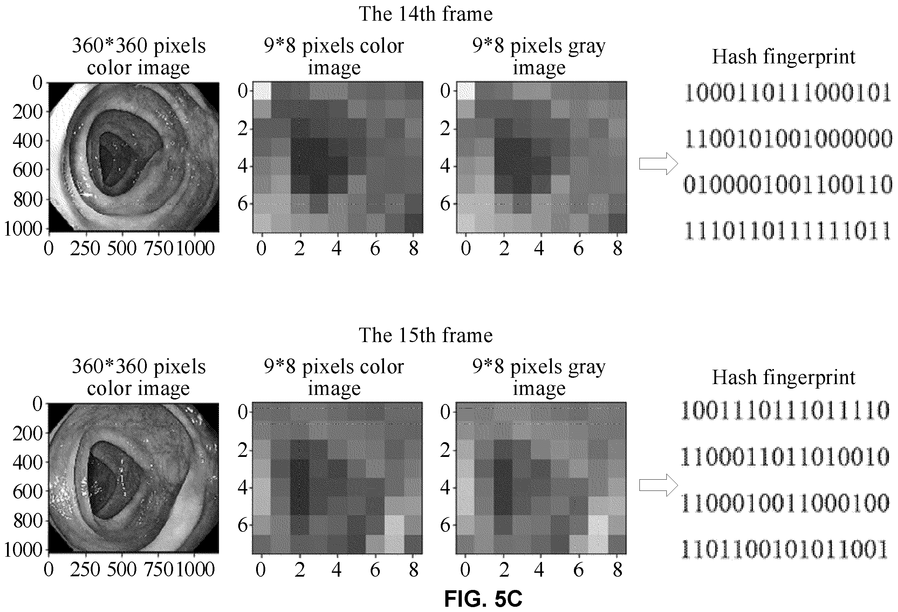

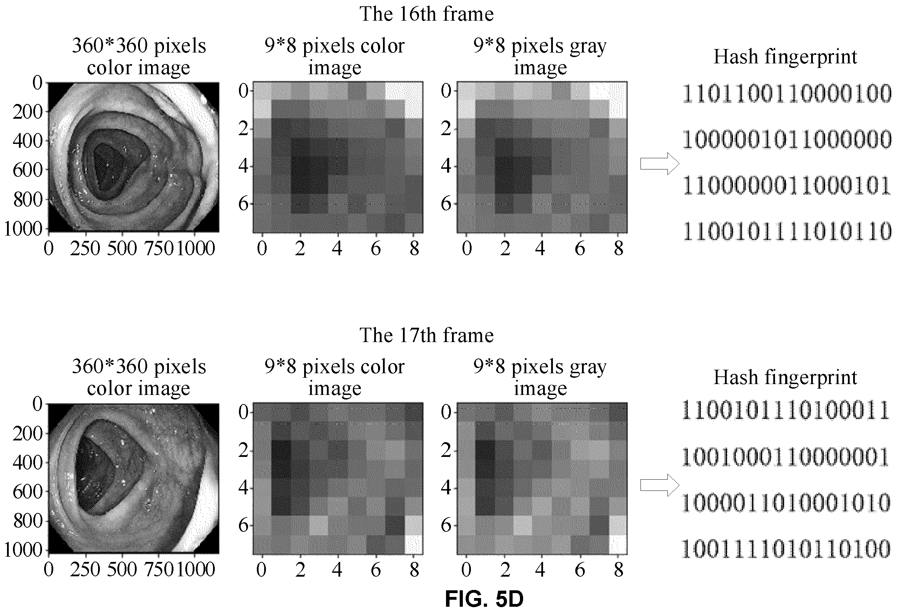

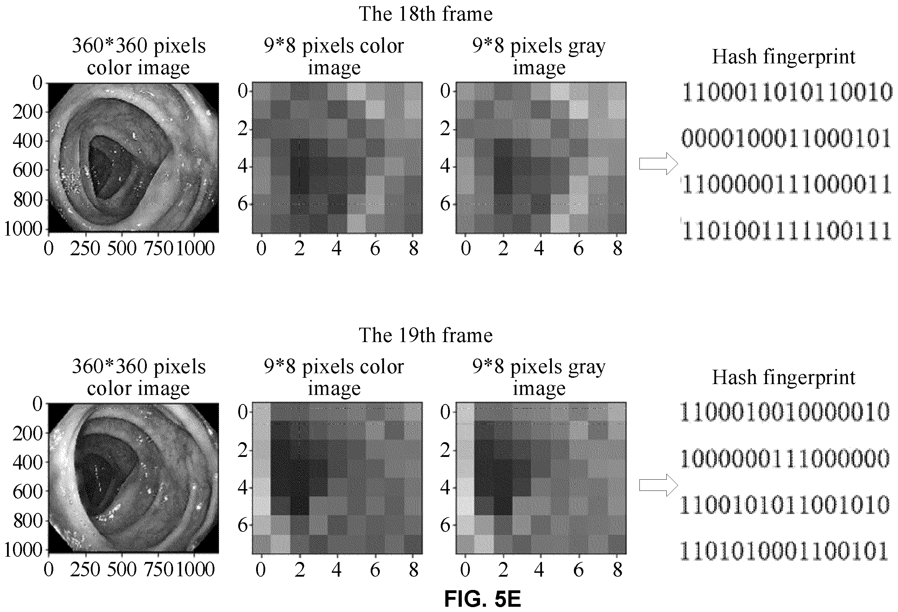

The embodiment further details the steps involved in the method of Example 1 for monitoring a colonoscopy withdrawal speed based on computer vision, as shown in FIGS. 5A-5E, comprising:

S1: Endoscopic equipment is used to acquire a colonoscopy video of up to 6 minutes in length, followed by decoding the video into images at a speed of two frames per second. Each image is cropped into a size having 360 × 360 pixels, further obtaining 720 images with a resolution of 360 × 360 pixels. The images are down-sampled by bicubic (cubic convolution) interpolation, dropping resolution to 8 $\times$ 9 pixels and obtaining 720 images with a resolution of 72 pixels.

S2: The down-sampled images are converted to grayscale images: since people have different sensitivities to red color, green color and blue color, the weights of 0.30, 0.59, and 0.11 are assigned to the corresponding color of each pixel in the images to obtain the gray value of this pixel. The following formula is used to calculate the gray value:

$$Gray = 0.30 \times R + 0.59 \times G + 0.11 \times B$$

S3: Different hash algorithm (DHA) is used to obtain the Hash fingerprints of the images. The difference of two adjacent pixels in each row is assigned 1 when the latter pixel is smaller than the former, otherwise the difference is assigned 0. The following Hash fingerprints correspond to the 10th - 19th frame of one image:

dHash value (n = 10):
0011111100111111100010111010010100010001000010010001011110100111;

dHash value (n = 11):
0011001100010001001011000001010000000010000000000000010100000110 01;

dHash value (n = 12):
0001001100011001100010011000001110000111100001111000111110011111;

dHash value (n = 13):
0001011101001011001000010010000100001101000010110001001100101010 0;

dHash value (n = 14):
1000110111000101110010100100000001000010011001101110110111111011

dHash value (n = 15):
1001110111011110110001101101001011000100110001001101100101011001

dhash value (n = 16):
1101100110000100100000101100000011000000110001011100 1011110101 1 0;

dHash value (n = 17):
1100101110100011100100011000000110000110100010101001111010110100 0;

dHash value (n = 18):
1100011010110011 000001000110001011100000111000011110100111110011 1

dhash value (n = 19):
1100010010000010100000011100000011001010110010101101010001100101 01;

S4: The dHash values of the two images are converted to binary differences which are then subject to exclusive OR. The number of "1" in the result of the exclusive OR operation, i.e., the number of different digits, is counted. It is the Hamming distance (d(x,y)).

Hamming distance (10,19) = 36;

Hamming distance (11,19) = 34;

Hamming distance (12,19) = 34;

Hamming distance (13,19) = 33;

Hamming distance (14,19) = 27;

Hamming distance (15,19) = 27;

Hamming distance (16,19) = 24;

Hamming distance (17,19) = 20;

Hamming distance (18,19) = 18.

S5: The similarity between the 19th frame of the colonoscopy video and the 9 previous images:

$$Sim = \frac{100 \times (64 - d(x,y))}{64}$$.

Hamming distance (10,19) = 43.8;

Hamming distance (11,19) = 46.9;

Hamming distance (12,19) = 46.9;

Hamming distance (13,19) = 48.4;

Hamming distance (14,19) = 57.8;

Hamming distance (15,19) = 57.8;

Hamming distance (16,19) = 62.5;

Hamming distance (17,19) = 68.8;

Hamming distance (18,19) = 71.9.

S6: The weighted similarity of the 10th -19th frames of the colonoscopy video are respectively calculated by using the formula $\overline{n} = \sum_{?=1}^{9} \frac{i}{} \times Sim_?$:     : $Sim$(10-19) = 60.9.

S7: The weighted overlapping rate of the 10th -19th frames of the colonoscopy video are respectively converted into a stability coefficient by using the formula $ESim = 100 - \overline{Sim}$: E$Sim$(10-19) = 39.1.

S8: A real-time stability coefficient of the 19th frame (of 9.5th second) of the colonoscopy video is 39.1 within a withdrawal speed warning range (30, 45). The system gives a doctor a safety reminder that the colonoscopy is withdrawal in the withdrawal speed warning range, and the colonoscopy is advised to withdraw slowly.

The disclosure also provides a device for monitoring a colonoscopy withdrawal speed based on computer vision, the device comprising:

an image acquisition module, configured to acquire, by endoscopic equipment, a real-time video of colonoscopy, decode the video into images, crop the images, and down-sample the cropped images where the structural information of the images is retained;

a grayscale image conversion module, configured to convert the images comprising the structural information to grayscale images;

a Hash fingerprint acquisition module, configured to obtain the Hash fingerprints of the images;

a Hamming distance calculation module, configured to calculate the Hamming distance between different images;

a similarity calculation module, configured to compare the Hash fingerprints of a current colonoscopy image with n previous colonoscopy images, to obtain an overlapping rate of the current colonoscopy image with any one of the n previous colonoscopy images, thereby obtaining a similarity between the current colonoscopy image and the any one of the n previous colonoscopy images;

a weighted similarity calculation module, configured to calculate a weighted similarity of the images at a point in time t;

a stability coefficient conversion module, configured to convert the weighted overlapping rate at the point in time t into a stability coefficient;

a mean stability coefficient conversion module, configured to calculate a mean stability coefficient of the colonoscopy images within a period of time 0-t;

a colonoscopy video analysis module, configured to analyze the real-time video of colonoscopy to obtain a first boundary between a standard colonoscopy video and a sub-standard colonoscopy video and a second boundary between the sub-standard colonoscopy video and a low-quality colonoscopy video, respectively; and

a withdrawal speed feedback module, configured to monitor in real time a stability coefficient of a withdrawal by a physician performing the colonoscopy, and feed the stability coefficient to the physician, wherein: when the stability coefficient is less than the first boundary, indicating the withdrawal speed of the colonoscopy is within a normal range; when the stability coefficient is greater than the first boundary, indicating the withdrawal speed of the colonoscopy is not within the normal range; when the stability coefficient is between the first boundary and the second boundary, a warning signal is given; and when the stability coefficient is greater than the second boundary, an emergency alarm is given.

The specific implementations of the modules correspond to the steps and will not be repeated here.

**Claims**

1. A computer-implemented method for monitoring colonoscopy withdrawal speed based on computer vision, the method comprising:

   1) acquiring (S1), by endoscopic equipment, a real-time video of a colonoscopy, decoding the video into images, cropping the images, and down-sampling the cropped images wherein structural information of the images is retained;
   2) converting (S2) the down-sampled images comprising the structural information to grayscale images;
   3) obtaining (S3) Hash fingerprints of the grayscale images, wherein the Hash fingerprints are obtained through a Hash algorithm;
   4) calculating (S4) a Hamming distance between the Hash fingerprint of a current greyscale image of the colonoscopy and the Hash fingerprint of each of n immediately previous grayscale images of the colonoscopy;
   5) comparing (S5) the Hash fingerprints of the current greyscale image of the colonoscopy with the n immediately previous greyscale images of the colonoscopy using the corresponding calculated Hamming distances, to obtain an overlapping rate of the current greyscale image of the colonoscopy with any one of the n immediately previous greyscale images of the colonoscopy, thereby obtaining a similarity between the current greyscale image of the colonoscopy and the any one of the n immediately previous greyscale images of the colonoscopy;
   6) calculating (S6) a weighted similarity of the greyscale images at a point in time t for the current grayscale image of the colonoscopy as a weighted sum of the similarity between the current grayscale image of the colonoscopy and each of the n immediately previous grayscale images of the colonoscopy;
   7) converting (S7) the weighted similarity of the greyscale images at the point in time t for the current grayscale image of the colonoscopy into a stability coefficient;
   8) calculating (S8) a mean stability coefficient within a period of time 0-t, wherein the mean stability coefficient is the mean of stability coefficients at all points in time;
   9) analyzing (S9) the real-time video of a plurality of colonoscopies to obtain a first boundary between a standard colonoscopy video and a sub-standard colonoscopy video and a second boundary between the sub-standard colonoscopy video and a low-quality colonoscopy video, respectively; wherein the plurality of colonoscopies comprises standard colonoscopy videos, sub-standard colonoscopy videos, and low-quality colonoscopy video; the standard colonoscopy videos are colonoscopy videos with a withdrawal time of more than 6 minutes, the sub-standard colonoscopy videos are colonoscopy videos with a withdrawal time of 4-6 minutes, and the low-quality colonoscopy videos are colonoscopy videos with a withdrawal time of less than 4 minutes; and

10) monitoring (S10) in real time, according to the operations 1) to 8), a stability coefficient of a withdrawal by a physician performing the colonoscopy, and feeding the stability coefficient to the physician, wherein: when the stability coefficient is less than the first boundary, indicating the withdrawal speed of the colonoscopy is within a normal range; when the stability coefficient is greater than the first boundary, indicating the withdrawal speed of the colonoscopy is not within the normal range; when the stability coefficient is between the first boundary and the second boundary, a warning signal is given; and when the stability coefficient is greater than the second boundary, an emergency alarm is given.

2. The method of claim 1, wherein in 1), the cropped images are down-sampled by bicubic interpolation.

3. The method of claim 1, wherein in 2), the down-sampled images are converted to the grayscale images by the following equation: $Gray = 0.30 \times R + 0.59 \times G + 0.11 \times B$; wherein R, G and B represent information values of red color, green color and blue color, respectively.

4. The method of claim 1, wherein in 3), the Hash fingerprints of the grayscale images are obtained by difference Hashing (dHash).

5. The method of claim 1, wherein in 5), the overlapping rate of the current greyscale image of the colonoscopy with any one of the n immediately previous greyscale images of the colonoscopy is calculated by the following equation:

$$Sim = \frac{100 \times (64 - d(x,y))}{64}$$

, wherein d(x, y) represents the Hamming distance between different greyscale images, $d(x,y) = \Sigma\ x \oplus y$, x and y represent character strings corresponding to different greyscale images, respectively, referring to the Hash fingerprints of the greyscale images, and $\oplus$ represents exclusive OR.

6. The method of claim 1, wherein in 7), the weighted similarity of the greyscale images at the point in time t for the current grayscale image of the colonoscopy is converted into the stability coefficient by the following equation: $ESim = 100 - \overline{Sim}$, wherein $\overline{Sim}$ represents the weighted similarity of the greyscale images at the point in time t for the current grayscale image of the colonoscopy.

7. A device for monitoring colonoscopy withdrawal speed based on computer vision, the device comprising:
an image acquisition module, configured to acquire, by endoscopic equipment, a real-time video of a colonoscopy, decode the video into images, crop the images, and down-sample the cropped images wherein structural information of the images is retained:

a grayscale image conversion module, configured to convert the down-sampled images comprising the structural information to grayscale images;
a Hash fingerprint acquisition module, configured to obtain Hash fingerprints of the grayscale images, wherein the Hash fingerprints are obtained through a Hash algorithm;
a Hamming distance calculation module, configured to calculate a Hamming distance between Hash fingerprint of a current greyscale image of the colonoscopy and the Hash fingerprint of each of n immediately previous grayscale images of the colonoscopy;
a similarity calculation module, configured to compare the Hash fingerprints of the current greyscale image of the colonoscopy with the n immediately previous greyscale images of the colonoscopy using the corresponding calculated Hamming distances, to obtain an overlapping rate of the current greyscale image of the colonoscopy with any one of the n immediately previous greyscale images of the colonoscopy, thereby obtaining a similarity between the current greyscale image of the colonoscopy and the any one of the n immediately previous greyscale images of the colonoscopy;
a weighted similarity calculation module, configured to calculate a weighted similarity of the greyscale images at a point in time t for the current grayscale image of the colonoscopy as a weighted sum of the similarity between the current grayscale image of the colonoscopy and each of the n immediately previous grayscale images of the colonoscopy;
a stability coefficient conversion module, configured to convert the weighted similarity of the greyscale images at the point in time t for the current grayscale image of the colonoscopy into a stability coefficient;
a mean stability coefficient conversion module, configured to calculate a mean stability coefficient within a period of time 0-t, wherein the mean stability coefficient is the mean of stability coefficients at all points in time;
a colonoscopy video analysis module, configured to analyze the real-time video of a plurality of colonoscopies to obtain a first boundary between a standard colonoscopy video and a sub-standard colonoscopy video and a second boundary between the sub-standard colonoscopy video and a low-quality colonoscopy video, respectively; wherein the plurality of colonoscopies comprises standard colonoscopy videos, sub-standard colonoscopy videos, and low-quality colonoscopy video; the standard colonoscopy videos are colonoscopy videos with a withdrawal time of more than 6 minutes, the sub-standard colonoscopy video are colonoscopy videos with a withdrawal time of 4-6 minutes, and the low-quality colonoscopy videos are colonoscopy videos with a withdrawal time of less than 4 minutes; and

a withdrawal speed feedback module, configured to monitor in real time a stability coefficient of a withdrawal by a physician performing the colonoscopy, and feed the stability coefficient to the physician, wherein: when the stability coefficient is less than the first boundary, indicating the withdrawal speed of the colonoscopy is within a normal range; when the stability coefficient is greater than the first boundary, indicating the withdrawal speed of the colonoscopy is not within the normal range; when the stability coefficient is between the first boundary and the second boundary, a warning signal is given; and when the stability coefficient is greater than the second boundary, an emergency alarm is given.

8. The device of claim 7, wherein in the similarity calculation module, the overlapping rate of the current greyscale image of the colonoscopy with any one of the n immediately previous greyscale images of the colonoscopy is calculated by the following equation: $Sim = \frac{100 \times (64 - d(x,y))}{64}$, wherein d(x, y) represents the Hamming distance between different greyscale images, $d(x,y) = \Sigma\, x \oplus y$, x and y represent character strings corresponding to different greyscale images, respectively, referring to the Hash fingerprints of the greyscale images, and $\oplus$ represents exclusive OR.

9. The device of claim 7, wherein in the stability coefficient conversion module, the weighted similarity of the greyscale images at the point in time t for the current grayscale image of the colonoscopy is converted into the stability coefficient by the following equation: $ESim = 100 - \overline{Sim}$, wherein $\overline{Sim}$ represents the weighted similarity of the greyscale images at the point in time t for the current grayscale image of the colonoscopy.

## Patentansprüche

1. Computerimplementiertes Verfahren zum überwachen von Koloskopie-Rückzugsgeschwindigkeit basierend auf Computervision, das Verfahren umfassend:

1) Erfassen (S1), durch endoskopische Ausrüstung, eines Echtzeitvideo einer Koloskopie, Dekodieren des Videos in Bilder, Beschneiden der Bilder und Heruntertakten der beschnittenen Bilder, wobei Strukturinformationen der Bilder beibehalten wird;
2) Umwandeln (S2) der heruntergetakteten Bilder, umfassend die Strukturinformationen, in Graustufenbilder;
3) Erlangen von (S3) Hash-Fingerabdrücke der Graustufenbilder, wobei die Hash-Fingerabdrücke durch einen Hash-Algorithmus erlangt werden;
4) Berechnen (S4) einer Hamming-Distanz zwischen dem Hash-Fingerabdruck eines aktuellen Graustufenbilds der Koloskopie und dem Hash-Fingerabdruck von jedem der n unmittelbar vorhergehenden Graustufenbilder der Koloskopie;
5) Vergleichen (S5) der Hash-Fingerabdrücke des aktuellen Graustufenbilds der Koloskopie mit den n unmittelbar vorhergehenden Graustufenbildern der Koloskopie unter Verwendung der entsprechenden berechneten Hamming-Distanzen, um eine Überlappungsrate des aktuellen Graustufenbilds der Koloskopie mit einem beliebigen der n unmittelbar vorhergehenden Graustufenbilder der Koloskopie zu erlangen, wodurch eine Ähnlichkeit zwischen dem aktuellen Graustufenbild der Koloskopie und dem beliebigen einen der n unmittelbar vorhergehenden Graustufenbilder der Koloskopie erlangt wird;
6) Berechnen (S6) einer gewichteten Ähnlichkeit der Graustufenbilder zu einem Zeitpunkt t für das aktuelle Graustufenbild der Koloskopie als eine gewichtete Summe der Ähnlichkeit zwischen dem aktuellen Graustufenbild der Koloskopie und jedem der n unmittelbar vorhergehenden Graustufenbilder der Koloskopie;
7) Umwandeln (S7) der gewichteten Ähnlichkeit der Graustufenbilder zum Zeitpunkt t für das aktuelle Graustufenbild der Koloskopie in einen Stabilitätskoeffizienten;
8) Berechnen (S8) eines mittleren Stabilitätskoeffizienten innerhalb einer Zeitperiode 0-t, wobei der mittlere Stabilitätskoeffizient der Mittelwert der Stabilitätskoeffizienten zu allen Zeitpunkten ist;
9) Analysieren (S9) des Echtzeitvideos einer Vielzahl von Koloskopien, um eine erste Grenze zwischen einem Standard-Koloskopie-Video und einem Sub-Standard-Koloskopie-Video bzw. eine zweite Grenze zwischen dem Sub-Standard-Koloskopie-Video und einem minderwertigen Koloskopie-Video zu erlangen; wobei die Vielzahl von Koloskopien Standard-Koloskopie-Videos, Sub-Standard-Koloskopie-Videos und minderwertige Koloskopie-Videos umfasst; die Standard-Koloskopie-Videos Koloskopie-Videos mit einer Rückzugszeit von mehr als 6 Minuten sind, die Sub-Standard-Koloskopie-Videos Koloskopie-Videos mit einer Rückzugszeit von 4-6 Minuten sind und die minderwertigen Koloskopie-Videos Koloskopie-Videos mit einer Rückzugszeit von weniger als 4 Minuten sind; und

10) Überwachen (S10) in Echtzeit, gemäß den Vorgängen 1) bis 8), eines Stabilitätskoeffizienten eines Rückzugs durch einen Arzt, der die Koloskopie ausführt, und Weiterleiten des Stabilitätskoeffizienten an den Arzt, wobei: wenn der Stabilitätskoeffizient kleiner ist als der erste Grenzwert, dies angibt, dass die Rückzugsgeschwindigkeit der Koloskopie innerhalb eines normalen Bereichs ist; wenn der Stabilitätskoeffizient größer ist als der erste Grenzwert, dies angibt, dass die Rückzugsgeschwindigkeit der Koloskopie nicht innerhalb des normalen Bereichs liegt; wenn der Stabilitätskoeffizient zwischen dem ersten Grenzwert und dem zweiten Grenzwert liegt, ein Warnsignal gegeben wird; und wenn der Stabilitätskoeffizient größer ist als der zweite Grenzwert, ein Notfallalarm gegeben wird.

2. Verfahren nach Anspruch 1, wobei in 1) die beschnittenen Bilder durch bikubische Interpolation heruntergetaktet werden.

3. Verfahren nach Anspruch 1, wobei in 2) die heruntergetakteten Bilder durch die folgende Gleichung in Graustufenbilder umgewandelt werden: $Grau = 0{,}30 \times R + 0{,}59 \times G + 0{,}11 \times B$; wobei R, G und B Informationswerte von roter Farbe, grüner Farbe bzw. blauer Farbe darstellen.

4. Verfahren nach Anspruch 1, wobei in 3) die Hash-Fingerabdrücke der Graustufenbilder durch Differenz-Hashing (dHash) erlangt werden.

5. Verfahren nach Anspruch 1, wobei in 5) die Überlappungsrate des aktuellen Graustufenbilds der Koloskopie mit einem der n unmittelbar vorhergehenden Graustufenbilder der Koloskopie durch die folgende Gleichung berechnet wird: $Sim = \frac{100 \times (64 - d(x,y))}{64}$, wobei d(x, y) die Hamming-Distanz zwischen verschiedenen Graustufenbildern darstellt, $d(x,y) = \Sigma x \oplus y$, x und y Zeichenketten darstellen, die jeweils verschiedenen Graustufenbildern entsprechen und sich auf die Hash-Fingerprints der Graustufenbilder beziehen, und $\oplus$ ausschließlich OR darstellt.

6. Verfahren nach Anspruch 1, wobei in 7) die gewichtete Ähnlichkeit der Graustufenbilder zu dem Zeitpunkt t für das aktuelle Graustufenbild der Koloskopie in den Stabilitätskoeffizienten durch die folgende Gleichung umgewandelt wird: $ESim = 100 - \overline{Sim}$, wobei $\overline{Sim}$ die gewichtete Ähnlichkeit der Graustufenbilder zu dem Zeitpunkt t für das aktuelle Graustufenbild der Koloskopie darstellt.

7. Vorrichtung zum Überwachen der Koloskopie-Rückzugsgeschwindigkeit basierend auf Computervision, die Vorrichtung umfassend:

ein Bilderfassungsmodul, das konfiguriert ist, um durch endoskopische Ausrüstung ein Echtzeitvideo einer Koloskopie zu erfassen, das Video in Bilder zu dekodieren, die Bilder zu beschneiden und die beschnittenen Bilder herunterzutakten, wobei Strukturinformationen der Bilder beibehalten werden;
ein Graustufenbild-Umwandlungsmodul, das konfiguriert ist, um die heruntergetakteten Bilder, umfassend die Strukturinformationen, in Graustufenbilder umzuwandeln;
ein Hash-Fingerabdruck-Erfassungsmodul, das konfiguriert ist, um Hash-Fingerabdrücke der Graustufenbilder zu erlangen, wobei die Hash-Fingerabdrücke durch einen Hash-Algorithmus erlangt werden;
ein Hamming-Distanz-Berechnungsmodul, das konfiguriert ist, um eine Hamming-Distanz zwischen dem Hash-Fingerabdruck eines aktuellen Graustufenbilds der Koloskopie und dem Hash-Fingerabdruck von jedem der n unmittelbar vorhergehenden Graustufenbilder der Koloskopie zu berechnen;
ein Ähnlichkeitsberechnungsmodul, das konfiguriert ist, um die Hash-Fingerabdrücke des aktuellen Graustufenbilds der Koloskopie mit den n unmittelbar vorhergehenden Graustufenbildern der Koloskopie unter Verwendung der entsprechenden berechneten Hamming-Distanzen zu vergleichen, um eine Überlappungsrate des aktuellen Graustufenbilds der Koloskopie mit einem beliebigen der n unmittelbar vorhergehenden Graustufenbilder der Koloskopie zu erlangen, wodurch eine Ähnlichkeit zwischen dem aktuellen Graustufenbild der Koloskopie und dem beliebigen einen der n unmittelbar vorhergehenden Graustufenbilder der Koloskopie erlangt wird;

ein Berechnungsmodul für gewichtete Ähnlichkeit, das konfiguriert ist, um eine gewichtete Ähnlichkeit der Graustufenbilder zu einem Zeitpunkt t für das aktuelle Graustufenbild der Koloskopie als eine gewichtete Summe der Ähnlichkeit zwischen dem aktuellen Graustufenbild der Koloskopie und jedem der n unmittelbar vorhergehenden Graustufenbilder der Koloskopie zu berechnen;

ein Stabilitätskoeffizient-Umwandlungsmodul, das konfiguriert ist, um die gewichtete Ähnlichkeit der Graustufenbilder zu dem Zeitpunkt t für das aktuelle Graustufenbild der Koloskopie in einen Stabilitätskoeffizienten umzuwandeln;

ein Umwandlungsmodul für mittleren Stabilitätskoeffizienten, das konfiguriert ist, um einen mittleren Stabilitätskoeffizienten innerhalb einer Zeitspanne von 0-t zu berechnen, wobei der mittlere Stabilitätskoeffizient der Mittelwert der Stabilitätskoeffizienten zu allen Zeitpunkten ist;

ein Koloskopie-Video-Analysemodul, das konfiguriert ist, um das Echtzeitvideo einer Vielzahl von Koloskopien zu analysieren, um eine erste Grenze zwischen einem Standard-Koloskopie-Video und einem Sub-Standard-Koloskopie-Video bzw. eine zweite Grenze zwischen dem Sub-Standard-Koloskopie-Video und einem minderwertigen Koloskopie-Video zu erlangen; wobei die Vielzahl von Koloskopien Standard-Koloskopie-Videos, Sub-Standard-Koloskopie-Videos und minderwertige Koloskopie-Videos umfasst; die Standard-Koloskopie-Videos Koloskopie-Videos mit einer Rückzugszeit von mehr als 6 Minuten sind, die Sub-Standard-Koloskopie-Videos Koloskopie-Videos mit einer Rückzugszeit von 4-6 Minuten sind und die minderwertigen Koloskopie-Videos Koloskopie-Videos mit einer Rückzugszeit von weniger als 4 Minuten sind; und

ein Rückzugsgeschwindigkeit-Rückmeldemodul, das konfiguriert ist, um in Echtzeit einen Stabilitätskoeffizienten eines Rückzugs durch einen Arzt, der die Koloskopie durchführt, zu überwachen und den Stabilitätskoeffizienten an den Arzt weiterzuleiten, wobei: wenn der Stabilitätskoeffizient kleiner ist als der erste Grenzwert, dies angibt, dass die Rückzugsgeschwindigkeit der Koloskopie innerhalb eines normalen Bereichs ist; wenn der Stabilitätskoeffizient größer ist als der erste Grenzwert, dies angibt, dass die Rückzugsgeschwindigkeit der Koloskopie nicht innerhalb des normalen Bereichs liegt; wenn der Stabilitätskoeffizient zwischen dem ersten Grenzwert und dem zweiten Grenzwert liegt, ein Warnsignal gegeben wird; und wenn der Stabilitätskoeffizient größer ist als der zweite Grenzwert, ein Notfallalarm gegeben wird.

8. Vorrichtung nach Anspruch 7, wobei in dem Ähnlichkeitsberechnungsmodul die Überlappungsrate des aktuellen Graustufenbilds der Koloskopie mit einem der n unmittelbar vorhergehenden Graustufenbilder der Koloskopie durch die folgende Gleichung berechnet wird: $Sim = \frac{100 \times (64 - d(x,y))}{64}$ , wobei d(x, y) die Hamming-Distanz zwischen verschiedenen Graustufenbildern darstellt, d(x,y) = $\Sigma x \oplus y$, x und y Zeichenketten darstellen, die jeweils verschiedenen Graustufenbildern entsprechen und sich auf die Hash-Fingerprints der Graustufenbilder beziehen, und $\oplus$ ausschließlich OR darstellt.

9. Vorrichtung nach Anspruch 7, wobei in dem Stabilitätskoeffizient-Umwandlungsmodul die gewichtete Ähnlichkeit der Graustufenbilder zum Zeitpunkt t für das aktuelle Graustufenbild der Koloskopie durch die folgende Gleichung in den Stabilitätskoeffizienten umgewandelt wird: $ESim$ = 100 - $\overline{Sim}$, wobei $\overline{Sim}$ die gewichtete Ähnlichkeit der Graustufenbilder zum Zeitpunkt t für das aktuelle Graustufenbild der Koloskopie darstellt.

**Revendications**

1. Procédé mis en oeuvre par ordinateur permettant de surveiller la vitesse de retrait d'une coloscopie sur la base de la vision par ordinateur, le procédé comprenant :

1) l'acquisition (S1), au moyen d'un équipement endoscopique, d'une vidéo en temps réel d'une coloscopie, le décodage de la vidéo en images, le rognage des images et le sous-échantillonnage des images rognées, les informations structurelles des images étant conservées ;
2) la conversion (S2) des images sous-échantillonnées comprenant les informations structurelles en images en niveaux de gris ;
3) l'obtention (S3) des empreintes digitales de hachage des images en niveaux de gris, lesdites empreintes digitales de hachage étant obtenues par un algorithme de hachage ;
4) le calcul (S4) d'une distance de Hamming entre l'empreinte de hachage d'une image en niveaux de gris courante de la coloscopie et l'empreinte de hachage de chacune des n images en niveaux de gris immédiatement précédentes de la coloscopie ;
5) la comparaison (S5) des empreintes digitales de hachage de l'image en niveaux de gris courante de la coloscopie aux n images en niveaux de gris immédiatement précédentes de la coloscopie à l'aide des distances de Hamming calculées correspondantes, pour obtenir un taux de chevauchement de l'image en niveaux de gris courante de la coloscopie avec l'une quelconque des n images en niveaux de gris immédiatement précédentes de la coloscopie, ce qui permet d'obtenir une similarité entre l'image en niveaux de gris courante de la coloscopie et l'une quelconque des n images en niveaux de gris immédiatement précédentes de la coloscopie ;

6) le calcul (S6) d'une similarité pondérée des images en niveaux de gris à un instant t pour l'image en niveaux de gris courante de la coloscopie sous la forme d'une somme pondérée de la similarité entre l'image en niveaux de gris courante de la coloscopie et chacune des n images en niveaux de gris immédiatement précédentes de la coloscopie ;

7) la conversion (S7) de la similarité pondérée des images en niveaux de gris à l'instant t pour l'image en niveaux de gris courante de la coloscopie en coefficient de stabilité ;

8) le calcul (S8) d'un coefficient de stabilité moyen dans une période de temps de 0 à t, dans laquelle le coefficient de stabilité moyen est la moyenne des coefficients de stabilité à tous les instants ;

9) l'analyse (S9) de la vidéo en temps réel d'une pluralité de coloscopies afin d'obtenir une première limite entre une vidéo de coloscopie standard et une vidéo de coloscopie sous-standard et une seconde limite entre la vidéo de coloscopie sous-standard et une vidéo de coloscopie de faible qualité, respectivement ; ladite pluralité de coloscopies comprenant des vidéos de coloscopie standard, des vidéos de coloscopie sous-standard et une vidéo de coloscopie de faible qualité ; lesdites vidéos de coloscopie standard étant des vidéos de coloscopie avec un temps de retrait supérieur à 6 minutes, lesdites vidéos de coloscopie sous-standard étant des vidéos de coloscopie avec un temps de retrait de 4 à 6 minutes et lesdites vidéos de coloscopie de faible qualité étant des vidéos de coloscopie avec un temps de retrait inférieur à 4 minutes ; et

10) la surveillance (S10) en temps réel, conformément aux opérations 1) à 8), d'un coefficient de stabilité d'un retrait par un médecin effectuant la coloscopie, et la transmission du coefficient de stabilité au médecin : lorsque le coefficient de stabilité est inférieur à la première limite, ceci indiquant que la vitesse de retrait de la coloscopie se trouve dans une plage normale ; lorsque le coefficient de stabilité est supérieur à la première limite, ceci indiquant que la vitesse de retrait de la coloscopie ne se trouve pas dans la plage normale ; lorsque le coefficient de stabilité est compris entre la première limite et la seconde limite, un signal d'avertissement étant émis ; et lorsque le coefficient de stabilité est supérieur à la seconde limite, une alarme d'urgence étant émise.

2. Procédé selon la revendication 1, en 1), lesdites images rognées étant sous-échantillonnées par interpolation bicubique.

3. Procédé selon la revendication 1, en 2), lesdites images sous-échantillonnées étant converties en images en niveaux de gris par l'équation suivante : $Gris$ = 0,30 × R + 0,59 × G + 0,11 × B ; dans laquelle R, G et B représentent respectivement les valeurs d'information de la couleur rouge, de la couleur verte et de la couleur bleue.

4. Procédé selon la revendication 1, en 3), lesdites empreintes de hachage des images en niveaux de gris étant obtenues par différence de hachage (dHash).

5. Procédé selon la revendication 1, en 5), ledit taux de chevauchement de l'image en niveaux de gris courante de la coloscopie avec l'une quelconque des n images en niveaux de gris immédiatement précédentes de la coloscopie étant calculé par l'équation suivante : $Sim = \frac{100 \times (64 - d(x,y))}{64}$ , dans laquelle d(x,y) représente la distance de Hamming entre différentes images en niveaux de gris, $d(x,y) = \Sigma\, x \oplus y$, x et y représentent respectivement des chaînes de caractères correspondant à différentes images en niveaux de gris, qui se rapportent aux empreintes de hachage des images en niveaux de gris, et $\oplus$ représente un OU exclusif.

6. Procédé selon la revendication 1, en 7), ladite similarité pondérée des images en niveaux de gris à l'instant t pour l'image en niveaux de gris courante de la coloscopie étant convertie en coefficient de stabilité par l'équation suivante : $ESim = 100 - \overline{Sim}$, dans laquelle $\overline{Sim}$ représente la similarité pondérée des images en niveaux de gris à l'instant t pour l'image en niveaux de gris courante de la coloscopie.

7. Dispositif permettant de surveiller la vitesse de retrait d'une coloscopie sur la base de la vision par ordinateur, le dispositif comprenant :

un module d'acquisition d'images, configuré pour acquérir, au moyen d'un équipement endoscopique, une vidéo en temps réel d'une coloscopie, décoder la vidéo en images, rogner les images et sous-échantillonner les images rognées, les informations structurelles des images étant conservées ;

un module de conversion d'images en niveaux de gris, configuré pour convertir les images sous-échantillonnées comprenant les informations structurelles en images en niveaux de gris ;

un module d'acquisition d'empreintes digitales de hachage, configuré pour obtenir des empreintes digitales de hachage des images en niveaux de gris, lesdites empreintes digitales de hachage étant obtenues par un algorithme de hachage ;

un module de calcul de distance de Hamming, configuré pour calculer une distance de Hamming entre l'empreinte de hachage d'une image en niveaux de gris courante de la coloscopie et l'empreinte de hachage de chacune des n images en niveaux de gris immédiatement précédentes de la coloscopie ;

un module de calcul de similarité, configuré pour comparer les empreintes de hachage de l'image en niveaux de gris courante de la coloscopie aux n images en niveaux de gris immédiatement précédentes de la coloscopie à l'aide des distances de Hamming calculées correspondantes, pour obtenir un taux de chevauchement de l'image en niveaux de gris courante de la coloscopie avec l'une quelconque des n images en niveaux de gris immédiatement précédentes de la coloscopie, ce qui permet d'obtenir une similarité entre l'image en niveaux de gris courante de la coloscopie et l'une quelconque des n images en niveaux de gris immédiatement précédentes de la coloscopie ;

un module de calcul de similarité pondérée, configuré pour calculer une similarité pondérée des images en niveaux de gris à un instant t pour l'image en niveaux de gris courante de la coloscopie sous la forme d'une somme pondérée de la similarité entre l'image en niveaux de gris actuelle de la coloscopie et chacune des n images en niveaux de gris immédiatement précédentes de la coloscopie ;

un module de conversion de coefficient de stabilité, configuré pour convertir la similarité pondérée des images en niveaux de gris à l'instant t pour l'image en niveaux de gris courante de la coloscopie en coefficient de stabilité ;

un module de conversion de coefficient de stabilité moyen, configuré pour calculer un coefficient de stabilité moyen dans une période de temps de 0 à t, dans laquelle le coefficient de stabilité moyen est la moyenne des coefficients de stabilité à tous les instants ;

un module d'analyse vidéo de coloscopie, configuré pour analyser la vidéo en temps réel d'une pluralité de coloscopies afin d'obtenir une première limite entre une vidéo de coloscopie standard et une vidéo de coloscopie sous-standard et une seconde limite entre la vidéo de coloscopie sous-standard et une vidéo de coloscopie de faible qualité, respectivement ; ladite pluralité de coloscopies comprenant des vidéos de coloscopie standard, des vidéos de coloscopie sous-standard et une vidéo de coloscopie de faible qualité ; lesdites vidéos de coloscopie standard étant des vidéos de coloscopie avec un temps de retrait supérieur à 6 minutes, lesdites vidéos de coloscopie sous-standard étant des vidéos de coloscopie avec un temps de retrait de 4 à 6 minutes et les vidéos de coloscopie de faible qualité étant des vidéos de coloscopie avec un temps de retrait inférieur à 4 minutes ; et

un module de retour de vitesse de retrait, configuré pour surveiller en temps réel un coefficient de stabilité d'un retrait par un médecin réalisant la coloscopie, et transmettre le coefficient de stabilité au médecin : lorsque le coefficient de stabilité est inférieur à la première limite, ceci indiquant que la vitesse de retrait de la coloscopie se trouve dans une plage normale ; lorsque le coefficient de stabilité est supérieur à la première limite, ceci indiquant que la vitesse de retrait de la coloscopie ne se trouve pas dans la plage normale ; lorsque le coefficient de stabilité est compris entre la première limite et la seconde limite, un signal d'avertissement étant émis ; et lorsque le coefficient de stabilité est supérieur à la seconde limite, une alarme d'urgence étant émise.

8. Dispositif selon la revendication 7, dans le module de calcul de similarité, ledit taux de chevauchement de l'image en niveaux de gris courante de la coloscopie avec l'une quelconque des n images en niveaux de gris immédiatement précédentes de la coloscopie étant calculé par l'équation suivante : $Sim = \frac{100 \times (64 - d(x,y))}{64}$, dans laquelle d(x,y) représente la distance de Hamming entre différentes images en niveaux de gris, $d(x,y) = \Sigma\, x \oplus y$, x et y représentent respectivement des chaînes de caractères correspondant à différentes images en niveaux de gris, qui se rapportent aux empreintes de hachage des images en niveaux de gris, et $\oplus$ représente un OU exclusif.

9. Dispositif selon la revendication 7, dans le module de conversion de coefficient de stabilité, ladite similarité pondérée des images en niveaux de gris à l'instant t pour l'image en niveaux de gris courante de la coloscopie étant convertie en coefficient de stabilité par l'équation suivante : $ESim = 100 - \overline{Sim}$, dans laquelle $\overline{Sim}$ représente la similarité pondérée des images en niveaux de gris à l'instant t pour l'image en niveaux de gris courante de la coloscopie.

A real-time video of colonoscopy is acquired by endoscopic equipment, the video is decoded into images (two frames per second), and the images are cropped into a size having 360 × 360 pixels. | S1

The images are converted to gray images. | S2

The Hash fingerprints of the images are obtained. | S3

The hash fingerprints of a current colonoscopic image are compared with that of its 9 previous images, and to obtain the similarity of the current image with any one of its 9 previous image. | S4

The instantaneous similarity value of the colonoscopy image at the point in time t is calculated. | S5

A stability coefficient of the colonoscopy image at the point in time t is calculated, and a mean stability coefficient of the colonoscopy images within a period of time 0-t is calculated. | S6

Analyzing the real-time video of colonoscopy to obtain a first boundary between a standard colonoscopy videos and a sub-standard colonoscopy videos and a second boundary between the sub-standard colonoscopy videos and a low-quality colonoscopy videos | S7

Analyzing the real-time video of colonoscopy to obtain a first boundary between a standard colonoscopy videos and a sub-standard colonoscopy videos and a second boundary between the sub-standard colonoscopy videos and a low-quality colonoscopy videos | S8

According to the operations S1 to S7, the withdrawal speed is monitored and fed back to the endoscopists in real time during colonoscopy by calculating stability coefficient. A warning signal is given when the stability coefficient exceeds 30, and an emergency alarm is given when the stability coefficient exceeds 45. | S9

**FIG. 1**

FIG. 2

original image

final image

(i,j)

(i',j')

u

v

| | | | |
|---|---|---|---|
| P-1-1 | P0-1 | P1-1 | P2-1 |
| P-10 | P00 | P10 | P20 |
| P-11 | P01 | P11 | P21 |
| P-12 | P02 | P12 | P22 |

**FIG. 3**

Inputting colonoscopy video — S1

Decoding video into 360 × 360 pixels images by two frames per second — S2

Using bicubic interpolation to zoom out images to remove unnecessary details of the images, leave only basic information — S3

Zooming-out images to a size having 9 × 8 pixels — S4

**FIG. 4**

The 10th frame

360*360 pixels color image

9*8 pixels color image

9*8 pixels gray image

Hash fingerprint

0011111100111111

1000101110100101

0001000100001001

0001011110100111

The 11th frame

360*360 pixels color image

9*8 pixels color image

9*8 pixels gray image

Hash fingerprint

0011001100010001

0010110000010100

0000001000000000

0001010000011001

**FIG. 5A**

EP 3 848 891 B1

The 12th frame

360*360 pixels color image | 9*8 pixels color image | 9*8 pixels gray image

Hash fingerprint

0001001100011001

1000100110000011

1000011110000111

1000111110011111

The 13th frame

360*360 pixels color image | 9*8 pixels color image | 9*8 pixels gray image

Hash fingerprint

0001011101001011

0010000100100001

0000110100001011

0001001100101010

**FIG. 5B**

## The 14th frame

360*360 pixels color image

9*8 pixels color image

9*8 pixels gray image

Hash fingerprint

1000110111000101

1100101001000000

0100001001100110

1110110111111011

## The 15th frame

360*360 pixels color image

9*8 pixels color image

9*8 pixels gray image

Hash fingerprint

1001110111011110

1100011011010010

1100010011000100

1101100101011001

**FIG. 5C**

The 16th frame

360*360 pixels
color image

9*8 pixels color
image

9*8 pixels gray
image

Hash fingerprint

1101100110000100

1000001011000000

1100000011000101

1100101111010110

The 17th frame

360*360 pixels
color image

9*8 pixels color
image

9*8 pixels gray
image

Hash fingerprint

1100101110100011

1001000110000001

1000011010001010

1001111010110100

**FIG. 5D**

EP 3 848 891 B1

The 18th frame

| 360\*360 pixels color image | 9\*8 pixels color image | 9\*8 pixels gray image | Hash fingerprint |
|---|---|---|---|

Hash fingerprint

1100011010110010

0000100011000101

1100000111000011

1101001111100111

The 19th frame

| 360\*360 pixels color image | 9\*8 pixels color image | 9\*8 pixels gray image | Hash fingerprint |
|---|---|---|---|

Hash fingerprint

1100010010000010

1000000111000000

1100101011001010

1101010001100101

**FIG. 5E**

EP 3 848 891 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017201494 A1

**Non-patent literature cited in the description**

- Colometer: a real-time quality feedback system for screening colonoscopy. *World Journal of Gastroenterology,* vol. 18 (32), 4270-4277

- Su1346 colonoscopy withdrawal velocity and image clarity measurement as a novel patient-centric real-time quality indicator for screening colonoscopy. *Gastrointestinal Endoscopy,* vol. 75 (4